# EUROPEAN PATENT SPECIFICATION

(11) **EP 2 644 084 B1**
(45) Date of publication and mention of the grant of the patent: **25.03.2015**
(21) Application number: 13156981.6
(22) Date of filing: 27.02.2013
(51) Int. Cl.: A61B 1/12

(54) **Endoscope**
Endoskop
Endoscope

(30) Priority: 26.03.2012 JP 2012069235
(43) Date of publication of application: 02.10.2013
(73) Proprietor: Fujifilm Corporation, Minato-ku Tokyo (JP)
(72) Inventor: Takahashi, Kazuaki, Kanagawa (JP)
(74) Representative: Klunker . Schmitt-Nilson . Hirsch

(56) References cited:
- WO-A1-2010/064506

## Description

### BACKGROUND OF THE INVENTION

### 1. Field of the invention

The present invention relates to an endoscope comprising a heat dissipation mechanism of an image sensor.

### 2. Description Related to the Prior Art

Diagnoses and operations using endoscopes have been widely performed in medical field. The endoscope is provided with an insert section to be inserted into a body cavity of a patient and a handling section provided at a proximal end of the insert section. A distal portion of the insert section incorporates an image sensor for imaging a region of interest in the body cavity.

In the distal portion of the insert section, heat generated in the image sensor and the like accumulates and raises the temperature of the insert section. Recently, pixel number and speed of reading image signals have been increased due to a demand to improve image quality of endoscopic images. As a result, heat from the image sensor is increased. An excessive increase in the temperature of the distal portion due to the heat from the image sensor makes the operation of the image sensor unstable. This causes noise in an image signal from the image sensor, resulting in deterioration of the image quality. To prevent the temperature rise, the image sensor is provided with a heat dissipation mechanism. Various types of heat dissipation mechanisms are known.

For example, in an endoscope disclosed in Japanese Patent Laid-Open Publication No. 2009-296542, a large-sized heat dissipation member is provided to an image sensor through an insulating member. In an endoscope disclosed in Japanese Patent Laid-Open Publication No. 2011-200401, a heat dissipation member that is fixed to a forceps channel is provided to an image sensor through an insulating member. In an endoscope disclosed in U. S. Patent Application Publication No. 2010/0033559 (corresponding to Japanese Patent Laid-Open Publication No. 2010-035815), an image sensor is provided with a cooling element disposed parallel with the image sensor. In an endoscope disclosed in Japanese Patent Laid-Open Publication No. 2009-066118, piping for flowing cooling fluid is provided close to an image sensor. In an endoscope disclosed in Japanese Patent Laid-Open Publication No. 2010-279527, an image sensor contacts with high thermal conductive ceramic. In an endoscope disclosed in Japanese Patent Laid-Open Publication No. 2010-201023, a heat storage material is disposed close to an image sensor. The heat storage material absorbs heat due to latent heat of a phase change.

Document WO 2010 064 506 discloses an endoscope with the features of the preamble of claim 1.

In the above-described endoscopes, the heat dissipation mechanisms are composed of the large-sized members, which increase material cost. The large-sized heat dissipation mechanism makes the insert section of the endoscope large in diameter and heavy. To reduce physical stress on a patient, it is necessary to downsize and reduce weight of the heat dissipation mechanism while good heat dissipation performance is maintained.

### SUMMARY OF THE INVENTION

An object of the present invention is to provide an endoscope provided with a lightweight, downsized, and inexpensive heat dissipation mechanism.

To achieve the above and other objects, an endoscope of the present invention is provided with a heat dissipation substrate, a multi-core cable, and a connection member. The heat dissipation substrate is attached to the image sensor such that the heat dissipation substrate is parallel with an imaging surface of the image sensor. The heat dissipation substrate transmits heat from the image sensor. The multi-core cable is composed of signal lines for transmitting signals to and from the image sensor, first shield members covering the respective signal lines, and a second shield member for covering and holding the signal lines together. The second shield member has an electrically conductive layer. The connection member connects the heat dissipation substrate and the electrically conductive layer. The connection member transmits the heat, from the image sensor, from the heat dissipation substrate to the electrically conductive layer.

It is preferable that the endoscope further comprises a circuit board. The image sensor is attached to a surface of the circuit board, and the heat dissipation substrate is attached to the back of the circuit board.

It is preferable that the connection member is formed using one of paste containing metal particles, soldering, wire bonding, and tape bonding.

It is preferable that the heat dissipation substrate is a flexible heat dissipation substrate having a film base made from polymer and a metal layer formed on the film base. It is preferable that the metal layer is formed on each surface of the film base.

It is preferable that the circuit board and the heat dissipation substrate are bonded using paste containing metal particles or soldering.

It is preferable that the heat dissipation substrate is a ceramic heat dissipation substrate having high thermal conductive ceramic and a metal layer formed on the high thermal conductive ceramic. It is preferable that the metal layer is formed on each surface of the high thermal conductive ceramic.

It is preferable that the heat dissipation substrate includes the high thermal conductive layers of different types, and the heat dissipation substrate is adhered to the circuit board using an adhesive.

It is preferable that the high thermal conductive layers of different types are a first high thermal conductive layer having thermal conductivity and electrically insulating properties and a second high thermal conductive layer having thermal conductivity higher than the thermal conductivity of the first high thermal conductive layer.

It is preferable that the first high thermal conductive layer is attached to the back of the circuit board.

According to the present invention, the connection member connects the heat dissipation substrate provided to the image sensor and the electrically conductive layer of the second shield member of the multi-core cable. Thereby, the heat from the image sensor is dissipated to the outside of the endoscope through the heat dissipation substrate and the second shield member of the multi-core cable that extends to the outside of the endoscope. Because the heat dissipation mechanism does not employ a large-sized heavy member, the heat dissipation mechanism is lightweight and downsized, and produced with low manufacture cost.

### BRIEF DESCRIPTION OF THE DRAWINGS

The above and other objects and advantages of the present invention will be more apparent from the following detailed description of the preferred embodiments when read in connection with the accompanied drawings, wherein like reference numerals designate like or corresponding parts throughout the several views, and wherein:
Fig. 1 is a perspective view illustrating an endoscope system employing an endoscope according to the present invention;
Fig. 2 is a front view illustrating an end cover of a distal portion of an insert section of the endoscope;
Fig. 3 is a cross-sectional view illustrating a flexible tube portion of the insert section of the endoscope;
Fig. 4 is a cross-sectional view of the distal portion of the endoscope according to a first embodiment of the present invention;
Fig. 5 is a cross-sectional view of the distal portion of the endoscope according to a modified example of the first embodiment; and
Fig. 6 is a cross-sectional view of the distal portion of the endoscope according to a second embodiment of the present invention.

### DESCRIPTION OF THE PREFERRED EMBODIMENTS

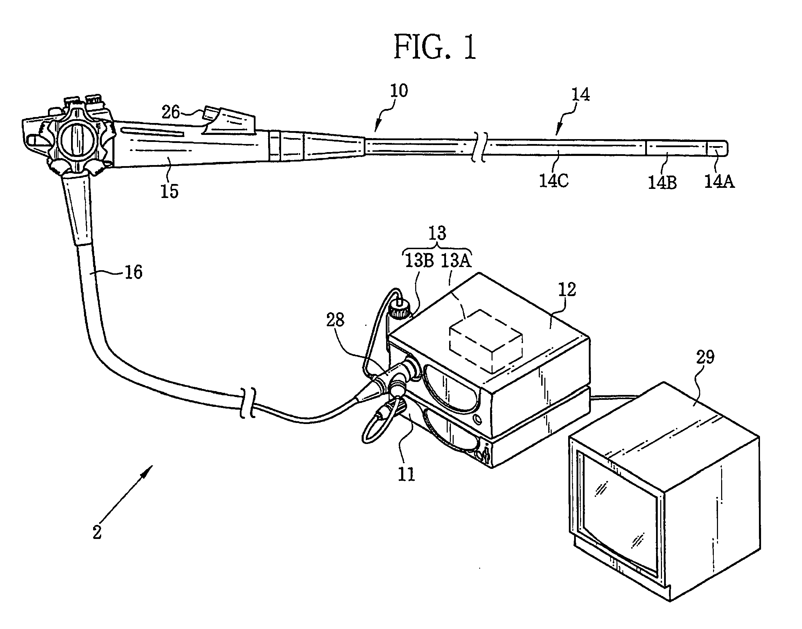

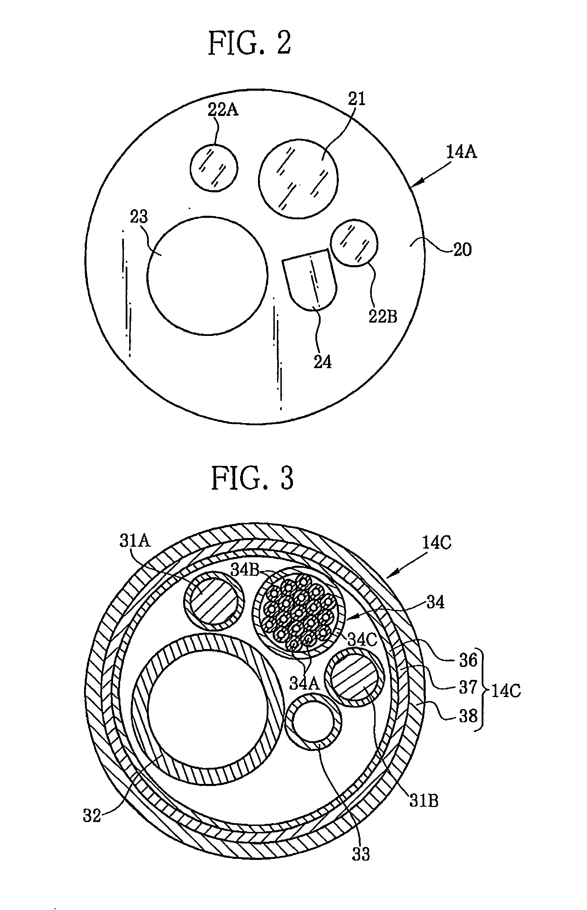

As shown in Figs. 1 and 2, an endoscope system 2 is composed of an endoscope 10, a processor device 11, a light source device 12, a monitor 29, and the like. The endoscope 10 is provided with an insert section 14 to be inserted into a body cavity of a patient, a handling section 15 connected to a basal (proximal) end portion of the insert section 14, and a universal cord 16 connected to the processor device 11 and the light source device 12. A connector 28 is connected to an end of the universal cord 16. The connector 28 is of a multi-connection type. The connector 28 is connected to each of the processor device 11 and the light source device 12.

An air/water feeding device 13 is incorporated in the light source device 12. The air/water feeding device 13 is composed of a well-known air-supply pump 13A and a water tank 13B. The air-supply pump 13A generates pressure to feed gas such as air and liquid such as cleaning water. The water tank 13B holds the cleaning water and is provided externally to the light source device 12.

The insert section 14 has a distal portion 14A, a bending portion 14B, and a flexible tube portion 14C. The distal portion 14A is provided with an imaging section for imaging the inside of the body cavity. The bending portion 14B is bendable. The flexible tube portion 14C has flexibility. Hereinafter, a distal end side of the insert section 14 is simply referred to as "the distal end side". A proximal end side of the insert section 14 is simply referred to as "the proximal end side".

An end cover 20 of the distal portion 14A is provided with a capture window 21, lighting windows 22A and 22B, a forceps outlet 23 from which forceps or the like are projected into the body cavity, and a jet nozzle 24. Behind the capture window 21, the imaging section is attached. The imaging section images the inside of the body cavity of the patient. The two lighting windows 22A and 22B are disposed symmetrically with respect to the capture window 21. The lighting windows 22A and 22B apply illumination light from the light source device 12 to a region of interest in the body cavity. The forceps outlet 23 is connected to a forceps inlet 26 provided to the handling section 15. A treatment tool such as forceps, an injection needle, or a high frequency surgical knife is inserted into the forceps inlet 26. The jet nozzle 24 ejects the air and the cleaning water, supplied from the air/water feeding device 13, to the capture window 21 to wash off dirt from the capture window 21 with the cleaning water and dry the capture window 21 with the air.

The processor device 11 performs various image processes on an image signal, inputted from the imaging section through the universal cord 16 and the connector 28, to produce an endoscopic image. The endoscopic image is displayed on the monitor 29 through a cable. The processor device 11 is connected to the light source device 12 through a communication cable, and communicates various types of control data with the light source device 12.

As shown in Fig. 3, light guides 31A and 31B, a forceps channel 32, an air/water channel 33, and a multi-core cable 34 run through the flexible tube portion 14C. The light guides 31A and 31B deliver the light from the light source device 12 to the lighting windows 22A and 22B. The forceps channel 32 is a flexible metal pipe and connects the forceps inlet 26 and the forceps outlet 23. The air/water channel 33 feeds the air and the cleaning water from the air/water feeding device 13 to the jet nozzle 24. The multi-core cable 34 electrically connects the processor device 11 and the imaging section.

The flexible tube portion 14C is composed of three layers, a helical tubular layer (flex) 36, a mesh tubular layer (blade) 37, and a resin (silicon rubber) layer 38 in this order from the inside. The helical tubular layer 36 is made from helically wound steel coils. The mesh tubular layer 37 covers the helical tubular layer 36 to prevent the helical tubular layer 36 from being stretched. The resin layer 38 has flexibility and covers the mesh tubular layer 37.

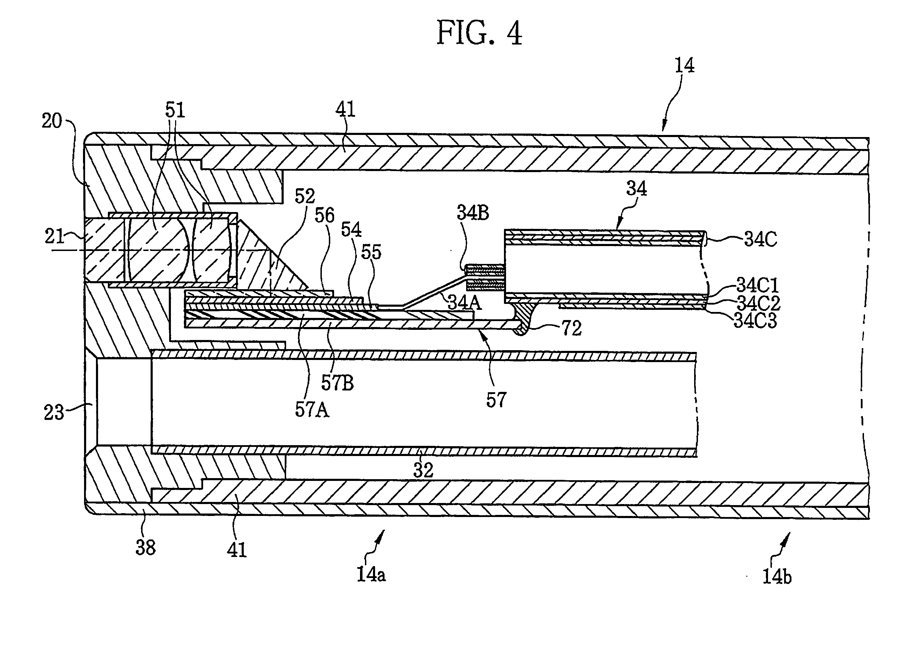

As shown in Fig. 4, a metal stationary tube 41 and the end cover 20 are provided inside the distal portion 14A. The stationary tube 41 has thermal conductivity and houses the forceps channel 32 and the imaging section. The end cover 20 fills gaps in an opening on the distal end side of the stationary tube 41. The stationary tube 41 and the end cover 20 are covered with the resin layer 38.

The light guides 31A and 31B, the forceps channel 32, the air/water channel 33, and the multi-core cable 34 run inside the stationary tube 41.

The forceps channel 32 is connected to the forceps outlet 23 provided through the end cover 20. Note that lighting lenses (not shown) are disposed behind the respective lighting windows 22A and 22B. Exits of the light guides 31A and 31B face the respective lighting lenses. The air/water channel 33 is connected to the jet nozzle 24. An end of each of the forceps channel 32, the light guides 31A and 31B, and the air/water channel 33 is fixed to the end cover 20. The other end of the forceps channel 32 is connected to the forceps inlet 26, and the other ends of the light guides 31A and 31B are connected to the light source device 12, and the other end of the air/water channel 33 is connected to the air/water feeding device 13, through the bending portion 14B, the flexible tube portion 14C, the handling section 15, and the like.

As shown in Fig. 3, the multi-core cable 34 is composed of signal lines 34A, first shield members 34B that cover the respective signal lines 34A, and a second shield member 34C. The second shield member 34C covers and holds the signal lines 34A, each covered with the first shield member 34B, together. Each of the first shield member 34B and the second shield member 34C functions as an electric shield layer and an electromagnetic shield layer. As shown in Fig. 4, the second shield member 34C is provided with an innermost layer 34C1, a middle layer 34C2, being an electrically conductive layer, and an outermost layer 34C3. Each of the innermost and outermost layers 34C1 and 34C3 is made from electrically insulating material. The middle layer 34C2 is made from electrically conductive material.

The distal portion of the endoscope 10 according to a first embodiment of the present invention incorporates the imaging section. As shown in Fig. 4, the imaging section is provided with an objective optical system 51, a prism 52, and an image sensor 54. Image light of the region of interest captured through the capture window 21 is incident on the prism 52 through the objective optical system 51. The prism 52 refracts the image light from the objective optical system 51 in a substantially vertical direction, and thereby forms an image of the region of interest on an imaging surface of the image sensor 54. The image sensor 54 is a CCD image sensor, a CMOS image sensor, or the like, and generates an image signal into which an image is converted photoelectrically. The image signal is outputted through a circuit board 55 provided on the opposite side of the imaging surface that is parallel (or substantially parallel) with a direction of insertion of the insert section 14. The circuit board 55 is electrically connected to each of the signal lines 34A of the multi-core cable 34. The image signal is sent to the processor device 11 through the multi-core cable 34. It is preferable that the size of the circuit board 55 is greater than or equal to the size of the image sensor 54. Transparent glass 56 protects an imaging surface side of the image sensor 54.

To dissipate the heat generated in the image sensor 54 to prevent malfunction of the image sensor 54, a heat dissipation substrate (thermal conductive substrate) 57 is overlaid onto the back of the circuit board 55. Note that, alternatively, the heat dissipation substrate 57 may be overlaid onto the back of the image sensor 54. The heat dissipation substrate 57 is provided with first and second high thermal conductive layers 57A and 57B so as to maintain electrical insulation with the circuit board 55 while having good thermal conductivity. In this embodiment, the first high thermal conductive layer 57A is made from electrically insulating material with relatively high thermal conductivity. The second high thermal conductive layer 57B is made from material with thermal conductivity higher than that of the first high thermal conductive layer 57A. The first and second high thermal conductive layers 57A and 57B are adhered to each other using an adhesive with good thermal conductivity. Note that the heat dissipation substrate 57 may be composed only of the first high thermal conductive layer 57A. When the electrically insulating properties are not necessary, the heat dissipation substrate 57 may be composed only of the second high thermal conductive layer 57B.

The first high thermal conductive layer 57A is adhered to the circuit board 55 disposed on and parallel (or substantially parallel) with the opposite side of the imaging surface of the image sensor 54 (the back of the image sensor 54) with the use of an electrically insulating adhesive. The adhesive preferably has high thermal conductivity in view of heat dissipation performance. It is preferable that a distal end side of the heat dissipation substrate 57 is flush with or protrudes relative to the circuit board 55. The proximal end side of the heat dissipation substrate 57 preferably protrudes relative to the circuit board 55. The proximal end side of the second high thermal conductive layer 57B preferably protrudes relative to the first high thermal conductive layer 57A.

A flexible heat dissipation substrate is used as the heat dissipation substrate 57. The flexible heat dissipation substrate is composed of a base layer and a metal layer formed on the base layer. The base layer is made from electrically insulating polymer with relatively high thermal conductivity, for example, polyimide. The metal layer is made from metal with high electrical conductivity. The base layer functions as the first high thermal conductive layer 57A. The metal layer functions as the second high thermal conductive layer 57B. For example, the base layer is made from polyimide. The metal layer is made from copper. A known product such as DIA-FINE (Japanese registered trademark No. 4901676) is a specific example of the flexible heat dissipation substrate.

A ceramic heat dissipation substrate may be used as the heat dissipation substrate 57. The ceramic heat dissipation substrate is composed of a base layer and a metal layer formed on the base layer. The base layer is made from electrically insulating ceramic with relatively high thermal conductivity. The metal layer is made from metal such as copper or aluminum. The base layer functions as the first high thermal conductive layer 57A. The metal layer functions as the second high thermal conductive layer 57B. For example, the base layer is made from alumina, aluminum nitride, or silicon nitride. Specific examples of the ceramic heat dissipation substrates include known products such as the above-mentioned ceramic metallized with the metal layer, DBC (direct-bond-copper, ceramic on which copper is bonded) (Japanese registered trademark No. 1877649), and DBA (direct-bond-aluminum, ceramic on which aluminum is bonded) (Japanese registered trademark No. 2011-082326).

Operation of the endoscope according to the first embodiment of the present invention is described. To perform an endoscopic examination, the insert section 14 of the endoscope 10 is inserted into the body cavity. During observation, the image sensor 54 is driven by a signal sent from the processor device 11 through the signal lines 34A. The signal lines 34A extend through the connector 28, the universal cord 16, the handling section 15, the flexible tube portion 14C, and the bending portion 14B to the image sensor 54. The image light of the region of interest is incident on the imaging surface of the image sensor 54 through the objective optical system 51 and the prism 52, and the image sensor 54 outputs the image signal. The image signal is transmitted to the processor device 11 in the reverse direction of the above-described transmission path of the signal from the processor device 11.

The image sensor 54 generates heat during operation. The heat is transmitted to the second high thermal conductive layer 57B with high thermal conductivity, through the first high thermal conductive layer 57A with relatively high thermal conductivity. The heat is transmitted from the distal end side to the proximal end side of the second high thermal conductive layer 57B. The heat is then transmitted to the middle layer 34C2 of the second shield member 34C of the multi-core cable 34 through a connection member 72. The heat transmitted to the middle layer 34C2 is transmitted through the multi-core cable 34, in the same direction as the image signal outputted from the image sensor 54. Eventually, the heat is released to the outside of the endoscope 10 through the universal cord 16.

It is preferable that the distal end side of the heat dissipation substrate 57 is flush with or protrudes relative to the image sensor 54 or the circuit board 55. It is preferable that the proximal end side of the heat dissipation substrate 57 protrudes relative to the image sensor 54 or the circuit board 55. Thereby, the capacity of the heat dissipation substrate 57 to receive the heat from the image sensor 54 increases. The second high thermal conductive layer 57B is preferably made from metal with high heat capacity, for example, copper. Thereby, the second high thermal conductive layer 57B can receive most of the heat generated by the image sensor 54 and transmitted through the first high thermal conductive layer 57A.

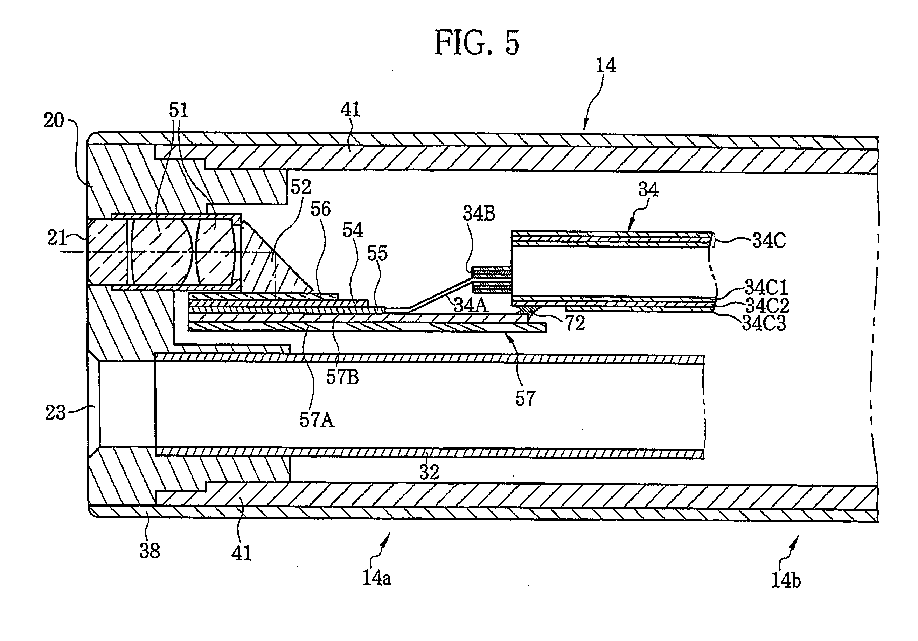

In a modified example of the first embodiment of the present invention, as shown in Fig. 5, the arrangement of the first high thermal conductive layer 57A and the second high thermal conductive layer 57B can be reversed only when an electrically insulating adhesive is used. Like reference numerals designate like or corresponding parts in Figs. 4 and 5, and descriptions thereof are omitted.

In the modified example of the first embodiment, it is necessary to prevent electrical connection between the signal lines 34A and the second high thermal conductive layer 57B made from the electrically conductive material. In the modified example, on the other hand, there is an advantage that the second high thermal conductive layer 57B made from the electrically conductive material is not electrically connected to the forceps channel 32 and the like because the first high thermal conductive layer 57A made from the electrically insulating material faces the forceps channel 32 and the like. Note that, also in this modified example, the heat dissipation substrate 57 is provided to the image sensor 54 through the circuit board 55 in a manner similar to the first embodiment. Alternatively, the heat dissipation substrate 57 may be adhered directly to the opposite side of the imaging surface of the image sensor 54 (the back of the image sensor 54) only using the electrically insulating adhesive.

As shown in Figs. 4 and 5, the connection member 72 thermally connects a proximal end portion of the second high thermal conductive layer 57B and a part of the middle layer 34C2 of the second shield member 34C. To improve the thermal conductivity, it is preferable that the connection member 72 has good electrical conductivity. The connection member 72 is formed using metal paste, such as silver paste, soldering, wire bonding, or tape bonding, for example. Note that, the proximal end side of the second high thermal conductive layer 57B preferably protrudes relative to the first high thermal conductive layer 57A. Thereby, the connection member 72 is formed easily.

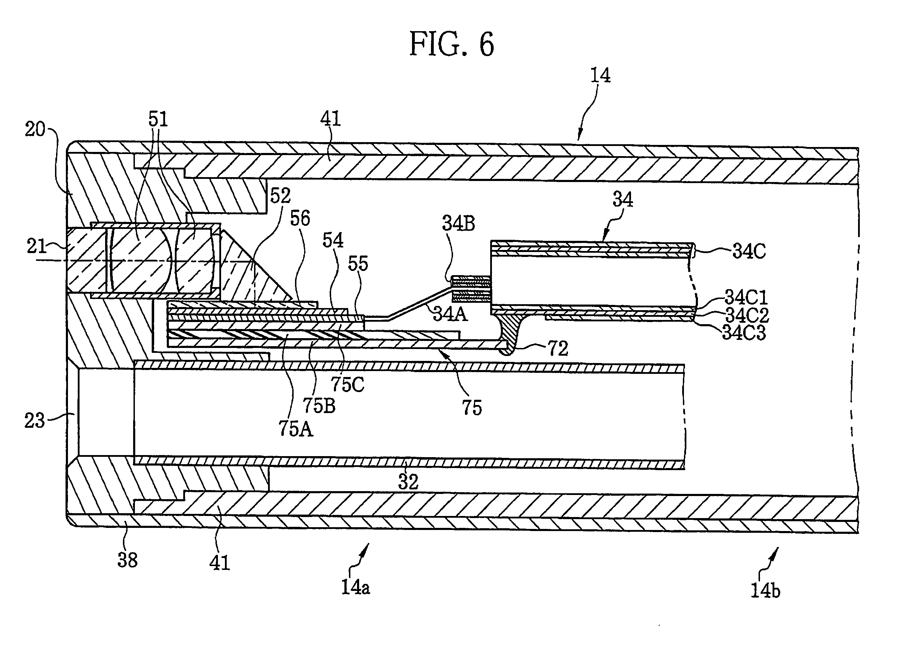

Next, referring to Fig. 6, an endoscope according to a second embodiment of the present invention is described. A heat dissipation substrate 75 is disposed on the opposite side of the imaging surface of the image sensor 54 through the circuit board 55 such that a plane direction of the heat dissipation substrate 75 is parallel with (or substantially parallel with) the image sensor 54. The heat dissipation substrate 75 is provided with first to third high thermal conductive layers 75A, 75B, and 75C. The first high thermal conductive layer 75A is sandwiched by the second and third high thermal conductive layers 75B and 75C. In this embodiment, the first high thermal conductive layer 75A is made from electrically insulating and thermally conductive material. Each of the second and third high thermal conductive layers 75B and 75C is made from material with good thermal conductivity. The first to third high thermal conductive layers 75A, 75B, and 75C are adhered together with an adhesive. Note that, like reference numerals designate like or corresponding parts in Figs. 4 and 6, and descriptions thereof are omitted.

The third high thermal conductive layer 75C is adhered to the circuit board 55 using an electrically insulating and thermally conductive adhesive, for example. When the surface of the circuit board 55, on the opposite side of the image sensor 54, is metallized, electrically conductive material such as solder or paste containing metal particles is preferably used for bonding the metallized surface of the circuit board 55 and the third high thermal conductive layer 75C in view of adhesive strength and adhesive reliability. Thereby, the heat is transmitted without using the adhesive layer. This is preferable in view of thermal conductivity, and thus the third high thermal conductive layer 75C receives the heat more effectively.

Similar to the heat dissipation substrate 57 of the first embodiment, it is preferable that the distal end side of the heat dissipation substrate 75 is in flush with or protrudes relative to the circuit board 55. It is preferable that the proximal end side of the heat dissipation substrate 75 protrudes relative to the circuit board 55. The proximal end side of the second high thermal conductive layer 75B preferably protrudes relative to the first high thermal conductive layer 75A. The proximal end side of the third high thermal conductive layer 75C is preferably shorter than the first high thermal conductive layer 75A.

As described in the first embodiment, a flexible heat dissipation substrate may be used as the heat dissipation substrate 75. The flexible heat dissipation substrate is provided with a base layer and two metal layers formed on respective surfaces of the base layer. The base layer and the two metal layers function as the first to third high thermal conductive layers 75A, 75B, and 75C, respectively. A ceramic heat dissipation substrate may be used as the heat dissipation substrate 75. The ceramic heat dissipation substrate is provided with a base layer and two metal layers formed on respective surfaces of the base layer. The base laser and the two metal layers function as the first to third high thermal conductive layers 75A, 75B, and 75C, respectively.

Similar to the first embodiment, the connection member 72 thermally connects a proximal end portion of the second high thermal conductive layer 75B and a part of the middle layer 34C2 of the second shield member 34C. The proximal end side of the third high thermal conductive layer 75C is preferably shorter than the first high thermal conductive layer 75A. Thereby, the connection member 72 is formed more easily. In this case, it is necessary to prevent the third high thermal conductive layer 75C from being electrically connected to the second high thermal conductive layer 75B and the signal lines 34A.

In the second embodiment, the third high thermal conductive layer 75C, in addition to the first and second high thermal conductive layers 75A and 75B, receives the heat from the image sensor 54. Thereby, higher heat dissipation performance is achieved.

The embodiments of the present invention are not limited to those described above.

## Claims

1. An endoscope (10) including an image sensor (54) provided in a distal portion (14A) of an insert section (14) to be inserted into a body cavity, the image sensor being disposed such that an imaging surface of the image sensor is parallel with a direction of insertion of the insert section, the endoscope comprising:
a heat dissipation substrate (57, 75) attached to the image sensor such that the heat dissipation substrate is parallel with the imaging surface, the heat dissipation substrate transmitting heat from the image sensor;
a multi-core cable (34) composed of signal lines (34A) for transmitting signals to and from the image sensor, first shield members (34B) covering the respective signal lines, and a second shield member (34C) for covering and holding the signal lines together, **characterized in that** the second shield member includes an electrically conductive layer (34C2); and
a connection member (72) for connecting the heat dissipation substrate (57, 75) and the electrically conductive layer (34C2), the connection member (72) transmitting the heat, from the image sensor (54), from the heat dissipation substrate (57, 75) to the electrically conductive layer (34C2).

2. The endoscope of claim 1, further comprises a circuit board (55), and the image sensor is attached to a surface of the circuit board, and the heat dissipation substrate is attached to a back of the circuit board.

3. The endoscope of claim 1 or 2, wherein the connection member is formed using one of paste containing metal particles, soldering, wire bonding, and tape bonding.

4. The endoscope of one of claims 1 to 3, wherein the heat dissipation substrate is a flexible heat dissipation substrate having a film base made from polymer and a metal layer formed on the film base.

5. The endoscope of claim 4, wherein the metal layer is formed on each surface of the film base.

6. The endoscope of one of claims 1 to 3, wherein the heat dissipation substrate is a ceramic heat dissipation substrate having high thermal conductive ceramic and a metal layer formed on the high thermal conductive ceramic.

7. The endoscope of claim 6, wherein the metal layer is formed on each surface of the high thermal conductive ceramic.

8. The endoscope of claim 5 or 7, wherein the circuit board and the heat dissipation substrate are bonded using paste containing metal particles or soldering.

9. The endoscope of one of claims 2 to 8, wherein the heat dissipation substrate includes the high thermal conductive layers of different types, and the heat dissipation substrate is adhered to the circuit board using an adhesive.

10. The endoscope of claim 9, wherein the high thermal conductive layers of different types are a first high thermal conductive layer having thermal conductivity and electrically insulating properties and a second high thermal conductive layer having thermal conductivity higher than the thermal conductivity of the first high thermal conductive layer.

11. The endoscope of claim 10, wherein the first high thermal conductive layer is attached to the back of the circuit board.

## Patentansprüche

1. Endoskop (10), enthaltend einen Bildsensor (84), der sich in einem distalen Bereich (14A) eines Einführabschnitts (14) zum Einführen in einen Körperhohlraum befindet, wobei der Bildsensor derart angeordnet ist, dass eine Bildgebungsfläche des Bildsensors parallel zu einer Einführrichtung des Einführabschnitts verläuft, umfassend:
ein Wärmeableitsubstrat (57, 75), angebracht an dem Bildsensor in der Weise,
dass das Wärmeableitsubstrat parallel zu der Bildgebungsfläche verläuft, wobei das Wärmeableitsubstrat Wärme aus dem Bildsensor überträgt;
ein Mehrkernkabel (34), bestehend aus Signalleitungen (34A) zum Übertragen von Signalen zu und von dem Bildsensor, erste Abschirmelemente (34B), die die betreffenden Signalleitungen bedecken, und ein zweites Abschirmelement (34C) zum Abdecken und Zusammenhalten der Signalleitungen, **dadurch gekennzeichnet, dass** das zweite Abschirmelement eine elektrisch leitende Schicht (34C2) enthält; und
ein Verbindungselement (72) das Wärmeableitsubstrat (57, 75) und die elektrisch leitende Schicht (34C2) verbindet, wobei das Verbindungselement (72) die Wärme von dem Bildsensor (54) aus dem Wärmeableitsubstrat (57, 75) zu der elektrisch leitenden Schicht (34C2) überträgt.

2. Endoskop nach Anspruch 1, weiterhin umfassend eine Schaltungsplatine (55), wobei der Bildsensor an einer Oberfläche der Schaltungsplatine befestigt ist und das Wärmeableitsubstrat auf der Rückseite der Schaltungsplatine befestigt ist.

3. Endoskop nach Anspruch 1 oder 2, bei dem das Verbindungselement mit Hilfe einer Metallpartikel enthaltenden Paste, durch Löten, durch Drahtbonden oder durch Tape-Bonden gebildet ist.

4. Endoskop nach einem der Ansprüche 1 bis 3, bei dem das Wärmeableitsubstrat ein flexibles Wärmeableitsubstrat mit einer Filmbasis aus einem Polymer und einer auf der Filmbasis gebildeten Metallschicht ist.

5. Endoskop nach Anspruch 4, bei dem die Metallschicht auf jeder Oberfläche der Filmbasis gebildet ist.

6. Endoskop nach einem der Ansprüche 1 bis 3, bei dem das Wärmeableitsubstrat ein keramisches Wärmeableitsubstrat mit einer stark thermisch leitenden Keramik und einer auf der stark thermisch leitenden Keramik gebildeten Metallschicht ist.

7. Endoskop nach Anspruch 6, bei dem die Metallschicht auf jeder Oberfläche der stark thermisch leitenden Keramik gebildet ist.

8. Endoskop nach Anspruch 5 oder 7, bei dem die Schaltungsplatine und das Wärmeableitsubstrat mit Hilfe einer Metallpartikel enthaltenden Paste oder durch Lot miteinander verbunden sind.

9. Endoskop nach einem der Ansprüche 2 bis 8, bei dem das Wärmeableitsubstrat stark thermisch leitende Schichten unterschiedlicher Typen enthält, und das Wärmeableitsubstrat mit Hilfe eines Klebstoffs an der Schaltungsplatine haftet.

10. Endoskop nach Anspruch 9, bei dem die stark thermisch leitenden Schichten unterschiedlicher Typen eine erste stark thermisch leitende Schicht mit Wärmeleitfähigkeit und elektrischen Isoliereigenschaften bzw. eine zweite stark thermisch leitende Schicht mit Wärmeleitfähigkeit größer als der Wärmeleitfähigkeit der ersten stark thermisch leitenden Schicht sind.

11. Endoskop nach Anspruch 10, bei dem die erste stark thermisch leitende Schicht an der Rückseite der Schaltungsplatine befestigt ist.

## Revendications

1. Endoscope (10) incluant un capteur d'image (54) prévu dans une partie distale (14A) d'une section d'introduction (14) à introduire dans une cavité corporelle, le capteur d'image étant disposé de telle sorte qu'une surface d'imagerie du capteur d'image soit parallèle à une direction d'introduction de la section d'introduction, l'endoscope comprenant :
un substrat dissipant la chaleur (57,75), fixé sur le capteur d'image de telle sorte que le substrat dissipant la chaleur soit parallèle à la surface d'imagerie, le substrat dissipant la chaleur transmettant la chaleur provenant du capteur d'image ;
un câble multiconducteur (34) composé de lignes signal (34A) destinées à transmettre des signaux à destination et en provenance du capteur d'image, des premiers éléments formant écran (34B) couvrant les lignes signal respectives, et un second élément faisant écran (34C) destiné à couvrir et maintenir les lignes signal ensemble, **caractérisé en ce que** le second élément formant écran inclut une couche conductrice de l'électricité (34C2), et
un élément de connexion (72) destiné à connecter le substrat dissipant la chaleur (57, 75) et la couche conductrice de l'électricité (34C2), l'élément de connexion (72) transmettant la chaleur, provenant du capteur d'image (54), du substrat dissipant la chaleur (57, 75) à la couche conductrice de l'électricité (34C2).

2. Endoscope selon la revendication 1, comprenant en outre une carte de circuit imprimé (55), et le capteur d'image est fixé à une surface de la carte de circuit imprimé, et le substrat dissipant la chaleur est fixé à un dos de la carte de circuit imprimé.

3. Endoscope selon la revendication 1 ou 2, dans lequel l'élément de connexion est formé à l'aide d'un élément parmi une pâte contenant des particules métalliques, un brasage, un microcâblage, et un soudage de bande.

4. Endoscope selon l'une quelconque des revendications 1 à 3, dans lequel le substrat dissipant la chaleur est un substrat dissipant la chaleur souple présentant une base de film fabriquée en polymère et une couche métallique formée sur la base de film.

5. Endoscope selon la revendication 4, dans lequel la couche métallique est formée sur chaque surface de la base de film.

6. Endoscope selon l'une quelconque des revendications 1 à 3, dans lequel le substrat dissipant la chaleur est un substrat dissipant la chaleur en céramique présentant une céramique de conductivité thermique élevée et une couche métallique formée sur la céramique de conductivité thermique élevée.

7. Endoscope selon la revendication 6, dans lequel la couche métallique est formée sur chaque surface de la céramique de conductivité thermique élevée.

8. Endoscope selon la revendication 5 ou 7, dans lequel la carte de circuit imprimée et le substrat dissipant la chaleur sont collés à l'aide d'une pâte contenant des particules métalliques ou d'un brasage.

9. Endoscope selon l'une quelconque des revendications 2 à 8, dans lequel le substrat dissipant la chaleur inclut les couches de conductivité thermique élevée de différents types, et le substrat dissipant la chaleur est collé à la carte de circuit imprimé à l'aide d'un adhésif.

10. Endoscope selon la revendication 9, dans lequel les couches de conductivité thermique élevée de différents types sont une première couche de conductivité thermique élevée présentant une conductivité thermique et des propriétés isolantes sur le plan électrique, et une seconde couche de conductivité thermique élevée présentant une conductivité thermique plus élevée que la conductivité thermique de la première couche de conductivité thermique élevée.

11. Endoscope selon la revendication 10, dans lequel la première couche de conductivité thermique élevée est fixée au dos de la carte de circuit imprimé.
